(19) Europäisches Patentamt
European Patent Office
Office européen des brevets

(11) **EP 4 310 177 A1**

(12) **EUROPEAN PATENT APPLICATION**

(43) Date of publication:
**24.01.2024 Bulletin 2024/04**

(21) Application number: **22185869.9**

(22) Date of filing: **19.07.2022**

(51) International Patent Classification (IPC):
***C12N 5/0735*** (2010.01)  ***C12N 5/077*** (2010.01)
***C12N 5/071*** (2010.01)  ***C12N 5/079*** (2010.01)

(52) Cooperative Patent Classification (CPC):
**C12N 5/0606; C12N 5/0012; C12N 5/0618;
C12N 5/0657; C12N 5/0689;** C12N 2500/25;
C12N 2501/115; C12N 2501/15; C12N 2501/155;
C12N 2501/415; C12N 2501/727; C12N 2506/02;
C12N 2513/00; C12N 2521/00; C12N 2533/90;

(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Medizinische Hochschule Hannover
30625 Hannover (DE)**

(72) Inventors:
• **Zweigerdt, Dr., Robert
30625 Hannover (DE)**

• **Drakhlis, Dr., Lika
30625 Hannover (DE)**
• **Manstein, Felix
30625 Hannover (DE)**
• **Dardano, Dr., Miriana
30625 Hannover (DE)**

(74) Representative: **Taruttis, Stefan Georg
TARUTTIS Patentanwaltskanzlei
Aegidientorplatz 2b
30159 Hannover (DE)**

(54) **PROCESS FOR PRODUCING ORGANOIDS FROM MAMMALIAN CELLS**

(57)    In vitro process for producing organoids from mammalian cells, the process comprising or the steps of cultivating pluripotent stem cells (PSC) in cell culture medium in a stirred tank bioreactor under conditions suitable for producing PSC aggregates, removing PSC aggregates suspended in cell culture medium from the bioreactor, and preferably in a flow channel, encapsulating PSC aggregates separately in biocompatible hydrogel to produce separate hydrogel-encapsulated PSC aggregates, incubating the hydrogel-encapsulated PSC aggregates in cultivation vessels under static conditions in medium containing at least one differentiation factor.

EP 4 310 177 A1

(52) Cooperative Patent Classification (CPC): (Cont.)
    C12N 2537/10

**Description**

The present invention relates to an in vitro process for automated production of organoids from mammalian pluripotent stem cells that are produced in suspension, preferably by cultivation in a stirred tank bioreactor.

The process for producing organoids has the advantage of automation of at least some, preferably of all steps of manipulation, and allows the production of a large number of organoids, e.g. from one batch of cells cultivated in one stirred tank bioreactor.

As an alternative, PSC can be replaced by non-human omnipotent or totipotent stem cells. Accordingly, in the process the stem cells can optionally be at least pluripotent stem cells. Generally, the PSC are generated without use of a human embryo. Preferably, pluripotent stem cells (PSC) are human PSC (hPSC), e.g. induced PSC (iPSC), e.g. generated from a mammalian cell sample, e.g. a blood or tissue sample, especially a human induced PSC (hiPSC), or an embryonic stem cell line (ESC), which preferably is non-human, or a human embryonic stem cell line (hESC). Generally, the PSC or ESC are not generated using a human embryo. The PSC preferably are a cell line, e.g. the hESC HES3.

Cultivated pluripotent stem cells (PSC) preferably are characterized by presence of the markers Tra-1-60 and SSEA4.

**State of art**

The trademark Matrigel (Corning Life Sciences and BD Bioscience) is known to designate a gelatinous matrix secreted by Engelbreth-Holm-Swarm (EHS) mouse sarcoma cells.

Lancaster et al. (Nature 501, 373-379 (2013)) describe the production of brain organoids from human PSCs under static conditions.

Richards et al., Biomaterials 112-123 (2017) describe the fabrication of cardiac organoids by depositing cardiomyocytes, cardiac ventricular fibroblast cells, umbilical vein endothelial cells (HUVEC), human adipose-derived microvascular endothelial cells (HAMEC), and human adipose-derived stem cells (hADSC) in agarose moulds to first generate spherical microtissues which are then assembled to spheroids.

Elliott et al. (2011) "NKX2-5(eGFP/w) hESCs for isolation of human cardiac progenitors and cardiomyocytes", Nature methods 8 (12), S. 1037-1040. DOI: 10.1038/nmeth.1740 describe the generation of hESC3 NKX2.5-eGFP cells.

US 2017/0198256 A1 describes that hiPSC were cultivated and harvested as single cells or cell clumps, which were combined with a liquid, cross-linkable PEG fibrinogen precursor solution to generate a statistical distribution of single hiPSC or clumps of hiPSC in the cross-linkable solution, which was cross-linked in forms to give cylinders of 6 mm width and 200 μm thickness. After cultivating these cylinders for 3 days, medium was changed to induce differentiation of the hiPSC, generating cardiac myocytes.

US 2016/0271183 A1 describes the differentiation of human embryonic stem cells in suspension in medium containing Matrigel with BMP4, Rho kinase inhibitor, activin-A and IWR-1 to cardiomyocytes.

WO 2019/174879 A1 describes a process for producing cardiac organoids by forming aggregates from cultivated pluripotent stem cells from a suspension by centrifuging the cells in a vessel having a U-shaped bottom, then incubating the cells localized at the bottom of the vessel under a medium under cell culture conditions, then embedding the cells within hydrogel and incubating the cells embedded within the hydrogel for solidifying the hydrogel, and covering the cells embedded in the solidified hydrogel with a second cell culture medium and incubating under cell culture conditions, followed by medium changes for differentiation of the cells.

A disadvantage of processes of prior art, e.g. WO 2019/174879 A1, is that the initial step requires that cells that are in suspension need to be formed into a shape by depositing and centrifuging cells in a U-bottom vessel.

**Object of the invention**

It is an object of the invention to provide an alternative process for producing organoids that can produce larger numbers of organoids, and which requires a lower number of processing steps or processing steps that can be automated. A preferred object is to provide a process that allows production of a large number of organoids without requiring shaping of suspended cells prior to embedding. Further preferable, the process shall allow automated control of the cultivation of cells prior to embedding.

**Description of the invention**

The invention achieves the object by an in vitro process for producing organoids from mammalian cells, the process comprising or essentially consisting of the steps of

a) cultivating pluripotent stem cells (PSC) in cell culture medium, for 2 to 3 days, which medium optionally is devoid of differentiation factors, with agitation of the medium, preferably providing the agitation in a stirred tank bioreactor, under conditions suitable for producing PSC aggregates, preferably generating PSC aggregates within 2 days of cultivation and optionally cultivating these aggregates for at least one subsequent day,

b) removing PSC aggregates suspended in cell culture medium from the bioreactor,

c) optionally selecting PSC aggregates having a pre-determined shape and/or size, and preferably in a flow channel, encapsulating PSC aggregates separately in biocompatible hydrogel to produce separate hydrogel-encapsulated PSC aggregates,

d) optionally selecting hydrogel-encapsulated PSC aggregates having a pre-determined shape and/or size, depositing hydrogel-encapsulated PSC aggregates in cultivation vessels, preferably each hydrogel-encapsulated PSC aggregate in a separate cultivation vessel, and

e) incubating the hydrogel-encapsulated PSC aggregates in cultivation vessels under static conditions in medium containing at least one differentiation factor,

wherein preferably the steps c) and d) are carried out while the PSC aggregates are moved, continuously or stepwise with stops between periods of movement, in a continuous flow channel.

Optionally, after the cultivation in a stirred-tank reactor for 2 to 3 days in medium devoid of differentiation factors of step a) for producing PSC aggregates, and before step b), the PSC aggregates are subsequently cultivated in the stirred-tank reactor in medium containing differentiation factors, preferably under the same agitation and oxygenation conditions, e.g. for another 1 to 7 days, preferably another 3 to 5 days.

The pluripotent stem cells preferably are human pluripotent stem cells (hPSC), e.g. induced hPSC (hiPSC).

The PSC aggregates produced by step a) preferably are sphere-shaped, e.g. in each dimension having a diameter of at least 100 $\mu$m, e.g. at least 200 $\mu$m or at least 300 $\mu$m, e.g. in each case up to 2 mm, up to 1.5 mm or up to 1 mm. It has been found that the cultivation under the conditions in a cell culture medium devoid of differentiation factors, also referred to as a first medium, produces PSC aggregates that consist of non-differentiated cells, and optionally aggregates that do not show a specific organization or arrangement of cells. Therefore, preferably prior to encapsulation and/or following encapsulation PSC aggregates are selected that are sphere-shaped and have a similar, e.g. within $\pm$100%, $\pm$80%, $\pm$60%, $\pm$50%, $\pm$40%, $\pm$30%, $\pm$20% or $\pm$10% size range, or have the same size.

The hydrogel can be on the basis of laminin and/or entactin and/or collagen and/or fibronectin and/or PEG, optionally on the basis of PEG (polyethylene glycol), e.g. in combination with fibronectin. The hydrogel preferably has a gelatinous structure. Preferably, the hydrogel is not cross-linkable, e.g. does not contain reactive groups that cross-link during the process, and accordingly, the process preferably is devoid of a step of cross-linking the hydrogel. Less preferred, the hydrogel may be cross-linkable, e.g. contain cross-linkable groups, and the process comprises a step of cross-linking the hydrogel. A preferred hydrogel is Matrigel.

The differentiation of the PSC aggregates is induced by differentiation factors only subsequent to encapsulating the PSC aggregates, by incubating the hydrogel-encapsulated PSC aggregates under static conditions in medium containing at least one differentiation factor, with subsequent change of medium, e.g. to a medium devoid of differentiation factor and/or subsequent change to a medium containing a second differentiation factor.

Preferably, e.g. for differentiating the encapsulated PSC aggregates, e.g. into cardiac organoids, the hydrogel-encapsulated PSC aggregates under static conditions are incubated in a second cell culture medium and incubating under cell culture conditions, preferably for 1 to 3 d, e.g. for 36 to 60 h,

removing the second medium from the cells and adding a third cell culture medium containing a first differentiation factor having activity to induce the WNT pathway and incubating under cell culture conditions for at least 6 h, e.g. for up to 3 d, preferably for 12 to 48 h, e.g. for 24h,

removing the third medium from the cells and adding a fourth cell culture medium not containing the first differentiation factor or containing an agent neutralizing the activity of the first differentiation factor, and incubating under cell culture conditions for at least 6 h, e.g. up to 3 d, preferably for 12 to 48 h, e.g. for 24 h,

removing the fourth medium from the cells and adding a fifth cell culture medium containing a second differentiation factor having activity to inhibit the WNT pathway and incubating under cell culture conditions for at least 1 d or at least 2 d, preferably for 46 to 50 h, e.g. for 48 h, wherein the third, fourth and fifth medium do not contain insulin,

removing the fifth medium from the cells and adding a sixth medium not containing insulin and not containing a differentiation factor having activity to induce or to inhibit the WNT pathway and incubating under cell culture conditions for at least 1 d or at least 2 d, preferably for 2 days,

removing the sixth medium from the cells and adding a seventh cell culture medium containing insulin and incubating under cell culture conditions for at least 1 d or at least 2 d, preferably for 2 days,

and subsequent change of the seventh medium for fresh cell culture medium at least every 2 days.

Generally preferred, during cultivation in the stirred-tank reactor, the medium is enriched E8 medium, and after removal of medium and aggregates from the reactor, the medium is changed to E8 medium, i.e. without enrichment.

In an embodiment, the differentiation can produce organoids, especially cardiac organoids, that are capable of generating blood. In this embodiment, the following specific cytokines are added in addition to differentiation factors for cardiac differentiation.

At encapsulation in hydrogel (day minus 2, d-2), the cells are kept in E8 medium and BMP4, e.g. to 10 ng/ml is added. After two days of incubation (day 0, d0), the medium is changed to RB- (without insulin) until day 7, from day 7 onwards, RB+ (with insulin) is used. At day 0 BMP4, e.g. to 10 ng/ml, and bFGF, e.g. to 5 ng/ml is added, together also referred to as miniboost, e.g. in addition to first differentiation factor CHIR. After 1 day of incubation, day 1 (d1), VEGF, e.g to 50 ng/ml, and bFGF, e.g. to 10 ng/ml is added, also referred to as boost, e.g. in medium devoid of differentiation factors. At day 3 of differentiation (d3), VEGF, e.g. to 50 ng/ml, bFGF, e.g. to 10 ng/ml, SCF, e.g. to 100 ng/ml, EPO, e.g. to 17 ng/ml, IL6, e.g. to 10 ng/ml, IL11, e.g. to 5 ng/ml, and IGF1, e.g. to 25 ng/ml, also referred to as boost 2, are added in addition to the second differentiation factor IWP2. At day 5 (d5) and at day 7 (d7) of differentiation, VEGF, e.g. to 50 ng/ml, bFGF, e.g. to 10 ng/ml, SCF, e.g. to 100 ng/ml, EPO, e.g. to 17 ng/ml, IL6, e.g. to 10 ng/ml, IL11, e.g. to 5 ng/ml, and IGF1, e.g. to 25 ng/ml, and TPO, e.g. to 30 ng/ml, FLT3, e.g. to 10 ng/ml, IL3, e.g. to 30 ng/ml, BMP4, e.g. to 10 ng/ml, and SHH, e.g. to 20 ng/ml, are added, e.g. also referred to as HEMCYT boost in addition to insulin-free medium (RB-) at d5 and in addition to insulin-containing medium (RB+) at d7.

In a specific embodiment, the invention also relates to organoids, especially cardiac organoids, that contain blood generating cells (BG) in heart-forming organoids (HFO), also referred to as BG-HFO, obtainable by the process for production using the specific cytokines during differentiation. These cardiac organoids that contain blood generating cells can be characterized by presenting the cardiac marker NKX2.5, the endothelial markers CD34 and CD144, and the hematopoietic markers CD43 and CD45.

In a further embodiment, the invention relates to organoids that contain lung progenitor cells that present NKX2.1, which organoids are produced by the process using differentiation factors during cultivation of the hydrogel-embedded PSC aggregates, e.g. CHIR, FGF10 BMP4. These organoids contain lung progenitor cells in addition to the cell types contained in the cardiac organoids (HFO), and accordingly, the organoids containing lung progenitor cells are also referred to as lung-HFO.

In a further embodiment, the invention relates to brain organoids, produced by the process for production using the specific cytokines during differentiation. These brain organoids can be characterized by neural epithelium morphology and by presenting neuronal markers SOX2 and TUJ1.

Generally, the cultivation conditions in the stirred tank bioreactor are a combination of cell culture medium devoid of differentiation factors in order to not induce differentiation of the PSC, and providing a sufficient dissolved oxygen concentration, and a stirrer speed that is sufficiently high for distributing dissolved oxygen throughout the medium volume and that is sufficiently low for maintaining cell aggregates, e.g. lower than the stirrer speed at which cell aggregates are dispersed into a suspension of single cells and/or lower than the stirrer speed at which cell viability is diminished.

For human PSC, preferred conditions that are suitable for producing PSC aggregates in cell culture medium with oxygen supply correspond to an agitation in a cylindrical bioreactor with a constant volumetric power input applied to the impeller of P/V = 0.13 to 1.0, e.g. P/V = 0.2 to 3.2, e.g. P/V = 0.26 $\pm$ 0.02 W/m$^3$, preferably P/V = 0.26 $\pm$ 0.01 W/m$^3$ or P/V = 0.26 W/m$^3$ of liquid contained in the bioreactor, calculated with equation 1

$$\text{equation 1:} \quad \frac{P}{V} = \frac{N^3 \times (D \times w \times b_n \div D_V \times \sin\theta)^5 \times \rho}{V}$$

wherein

P = Power [W]
V = liquid volume [m$^3$]
N = impeller rotational speed [s$^{-1}$]
D = impeller diameter [m]
w = impeller width [m]
$b_n$ = number blades of impeller [-]
$D_V$ = diameter of bioreactor [m]
$\theta$ = blade angle [°]
$\rho$ = liquid density [kg/m$^3$], wherein the liquid density is determined for the cell-free fraction of the liquid contained in the bioreactor.

In an exemplary bioreactor having a diameter $D_V$ of 0.064 m filled with 150 mL liquid, i.e. medium (density of

993.3 kg/m$^3$) including cells, with an impeller having a diameter of 0.034 m, a width of 0.01 m, a number of blades of 8, a blade angle of 60°, a volumetric power input P/V = 0.26 W/m$^3$ was obtained for 50 rpm.

Generally, oxygen supply preferably is bubble-free, e.g. provided by filling the reactor volume above the medium with oxygen, also known as overlay gassing, or by diffusion of oxygen through an oxygen-permeable membrane arranged in the bioreactor with air flow or oxygen flow applied to one side of the membrane.

Removing the PSC aggregates suspended in cell culture medium from the bioreactor in step b) can be through a tubing, optionally with overpressure applied to the bioreactor or with underpressure applied to the tubing.

Preferably, from the PSC aggregates removed from the bioreactor, PSC aggregates having a pre-determined shape and/or size are selected in order to exclude PSC aggregates that do not have a suitable shape and/or size, and optionally fulfil other criteria, from the process. Other criteria that may be used for excluding PSC aggregates e.g. are presence of solid particles in PSC aggregates, absence of certain cell-surface markers, presence of certain cell-surface markers, each determined by binding of a labelled antibody added to the medium surrounding the PSC aggregates, an optical density outside of a predetermined range under illumination with light.

Preferably, selecting of PSC aggregates having a sphere-shape and/or a size of 300 μm to 2 mm or up to 1.5 mm or up to 1 mm, optionally excluding PSC aggregates showing other criteria, is by optical detection of PSC aggregates under illumination with light, e.g. by recording for each PSC aggregate an image, light scatter, light transmission, each from at least one side using one detector, e.g. an electronic camera coupled to a computer for determining the size and/or shape of each aggregate, preferably using optical detection from at least two sides using at least two detectors directed from different angles, e.g. offset by 90°, at the same spot through which each of the PSC aggregates is guided, and comparing measurement results with pre-determined parameters. Generally, the size of the aggregates can be determined as the diameter in their largest extension.

Preferably, the PSC aggregates are guided singly in a continuous flow through a spot onto which at least one detector is directed, onto which preferably at least two detectors are directed. The PSC aggregates can be guided by flowing in suspension, e.g. in medium, through a first flow channel, which can be a microfluidic channel. In order to guide the PSC aggregates separately, i.e. as a file of single, separated PSC aggregates suspended in cell culture medium, the flow channel preferably has an inner diameter corresponding to the size of the PSC aggregates with a factor of 1.2 to 2.5 or a factor of up to 2.0 or up to 1.8. The first flow channel preferably is optically transparent at least at the spot onto which illumination and at least one detector, preferably at least two detectors are directed.

Generally, each flow channel can e.g. be a conduit arranged in a solid carrier, e.g. of optically transparent plastic or glass, or a tubing.

In step c), in a flow channel, PSC aggregates are encapsulated separately in biocompatible hydrogel to produce separate hydrogel-encapsulated PSC aggregates. The flow channel in which the PSC aggregates are each separately encapsulated in hydrogel is also referred to as a second flow channel. The PSC aggregates are each separately encapsulated by the PSC aggregates flowing separately in the second flow channel, and a hydrogel is introduced into the second flow channel, e.g. continuously. Alternatively, the hydrogel is introduced stepwise, preferably under the control of a detector, such that a portion of the hydrogel is introduced into the second flow channel during the passage of one of the PSC aggregates past the site at which the hydrogel portion is introduced. The site at which the hydrogel is introduced, also referred to as hydrogel injection site, can have one or more, preferably at least two or at least three, e.g. up to 6 or up to 5 or 4 injection openings arranged around the circumference of the second flow channel around one cross-section of the second flow channel. Preferably, the injection openings forming one hydrogel injection site are arranged at one common axial site and offset about the circumference of the second flow channel, especially in order to apply the hydrogel concurrently from at least two directions onto the same cross-sectional area of each PSC aggregate.

In step c), preferably prior to encapsulating, the PSC aggregates can be separated by diluting the suspension of PSC aggregates by addition of cell-compatible medium to a final concentration of PSC aggregates that provides their statistical distribution sufficient for separating the PSC aggregates in the first flow channel. Separation of PSC aggregates in the flow channel can e.g. be a distance corresponding to one-fold to ten-fold or up to fivefold or up to two-fold the average diameter of the PSC aggregates. Optionally, separation of PSC aggregates in flow channels can be by introducing a separator fluid into the flow channel, which separator fluid is non-miscible with the medium in which the PSC aggregates are suspended, e.g. non-miscible with the first medium. A separator fluid is preferably injected into the flow channel, e.g. at a site upstream of the hydrogel injection site, between two neighbouring PSC aggregates when passing, wherein passing of PSC aggregates can be detected by an optical sensor directed to the flow channel and controlling the injection of separator fluid in dependence on the sensor signal. An exemplary separator fluid is hydrophobic, e.g. an oil.

Preferably, the introduction of hydrogel into the second flow channel is controlled to be interrupted or stopped, when no PSC aggregate moves past the hydrogel injection site. Therein, the movement of PSC aggregates separately past the hydrogel injection site is at constant speed, alternatively, the movement of PSC aggregates may be stopped for a predetermined period when a PSC aggregate is positioned at the hydrogel injection site. Preferably, the flow channel has a constant inner diameter along its length that includes the first flow channel and the second flow channel and the third flow channel in order to minimize shear forces acting onto the PSC aggregates. Generally preferred, the first flow channel, the second flow channel and a third flow channel are connected to one another without changes in inner diameter at connections, preferably each flow channel having the same inner diameter, and further preferably the first, second and third flow channels are sections of one continuous flow channel.

The optional step of selecting hydrogel-encapsulated PSC aggregates having a pre-determined shape and/or size and/or fulfilling another criterion, can be generally be performed as described with reference to selecting PSC aggregates in step c), e.g. by optical detection of PSC aggregates under illumination with light, e.g. by recording for each PSC aggregate an image, light scatter, light transmission, each from at least one side using one detector, preferably from at least two sides using at least two detectors directed from different angles, e.g. offset by 90°, at the same spot through which each of the PSC aggregates is guided, and comparing measurement results with pre-determined parameters. In order to use detection of hydrogel that encapsulates a PSC aggregate, optionally the hydrogel can contain a dye, or a dye can be added to the medium flowing through the flow channel, especially added to the second flow channel upstream or downstream of the hydrogel injection site.

The step of depositing hydrogel-encapsulated PSC aggregates in separate cultivation vessels can be by relative movement of an outlet of a third flow channel along an arrangement of cultivation vessels, wherein the third flow channel is connected to the outlet of the second flow channel. The relative movement of the outlet of the third flow channel over an arrangement of cultivation vessels can be co-ordinated by a detector that is set-up for detecting presence of a PSC aggregate encapsulated in hydrogel, which detector is arranged at the second or third flow channel.

Optionally, after depositing hydrogel-encapsulated PSC aggregates in separate cultivation vessels, medium that has been deposited into the cultivation vessels may be removed, e.g. by aspiration.

The step of incubating the hydrogel-encapsulated PSC aggregates in separate cultivation vessels in medium containing at least one differentiation factor is by adding at least one differentiation factor, e.g. contained in a cell culture medium, to cultivation vessels. The at least one differentiation factor may comprise a first differentiation factor and a second differentiation factor, wherein the second differentiation factor is added to the cultivation medium present in the cultivation vessel or replaces the medium present in the cultivation vessel, e.g. after removal of the medium containing the first differentiation factor. Similarly, medium can be added to or exchanged in cultivation vessels, e.g. by pipetting, optionally aspirating the previously present medium.

Differentiation of hydrogel-embedded PSC aggregates, especially of human PSC, can be into cardiac cells in order to produce cardiac organoids.

For example, for differentiating PSC to cardiac cells, a first differentiation factor can have activity to induce the WNT pathway, preferably an inhibitor of GSK3beta (glycogen synthase kinase 3 beta), and preferably has no effect or cross-reactivity on CDKs (cyclin-dependent kinases), e.g. CHIR99021 (CHIR, 6-[[2-[[4-(2,4-dichlorophenyl)-5-(5-methyl-1H-imidazol-2-yl)-2 pyrimidinyl]amino]ethyl]amino]-3-pyridinecarbonitrile), e.g. at $7.5\mu M$, or followed by replacement for a second differentiation factor, e.g. second differentiation factor preferably is an inhibitor of the WNT pathway activator Porcupine, e.g. IWP2 (Inhibitor of WNT Production-2, CAS No. 686770-61-6), e.g. at a concentration of $5\mu M$.

The invention is now described in greater detail by way of an example and with reference to the figures, which show in

- Fig. 1 a schematic of a process of the invention,
- Fig. 2 pictures of differentiated cardiac organoids produced according to the invention,
- Fig. 3 pictures of differentiated cardiac organoids produced according to the invention,
- Fig. 4 schematic of process of the invention using differentiation supplemented with specific cytokines at specific time-points for producing blood generating organoids,
- Fig. 5 pictures of blood generating cardiac organoids produced according to the invention,
- Fig. 6 quantitative FACS analyses for CD31, CD144 and CD34,
- Fig. 7A cryosection of a lung-progenitor cell containing organoid (HFO) with immunocytochemical staining and DAPI staining,
- Fig. 7B a cryosection of a of a lung-progenitor cell containing organoid (HFO) with immunocytochemical staining.
- Fig. 8A brightfield image of a brain organoid,
- Fig. 8B cryosection of a brain organoid with immunocytochemical staining,
- Fig. 9A brightfield image of a brain organoid,
- Fig. 9B cryosection of a brain organoid with immunocytochemical staining

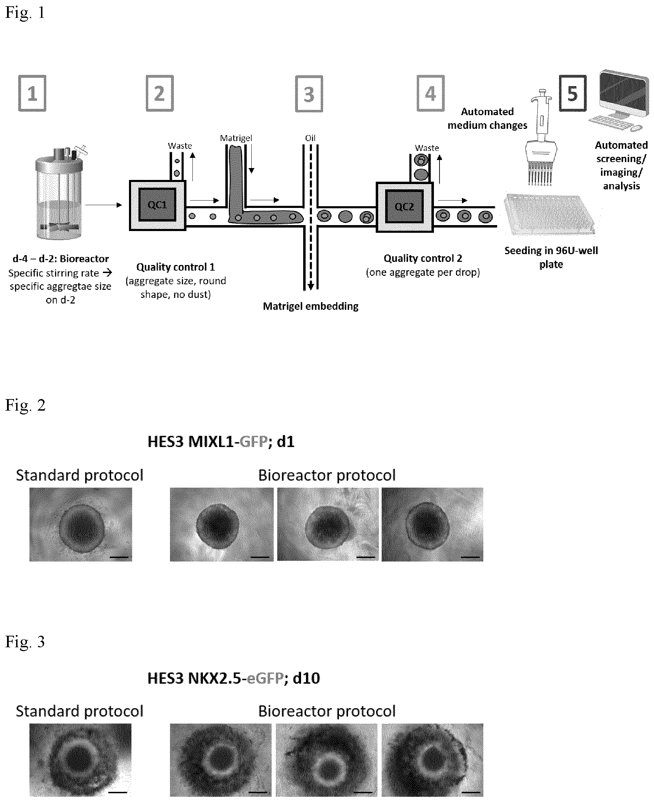

Fig. 1 depicts a stirred tank bioreactor, in which PSC, e.g. produced by a pre-culture, according to step a) are cultivated in a stirred tank bioreactor in medium without differentiation factor for day -4 to day -2, with formation of PSC aggregates occurring at day -3 (1), preferably with one additional day of cultivation for growth of PSC aggregates. The medium containing the PSC aggregates is removed from the bioreactor and introduced into a microfluidic flow channel, with the PSC aggregates being suspended in the medium at a concentration that statistically distributes the PSC aggregates to a file of separated single PSC aggregates in the flow channel. The flow channel comprised a first flow channel and a downstream connected second flow channel and a third flow channel. In the first flow channel, PSC aggregates were illuminated with light and, using a camera as an optical detector, PSC aggregates having a pre-determined size and sphere-shape were guided into the second flow channel, whereas aggregates having a size or shape outside the pre-determined values were discarded (waste) (2). The second flow channel has a hydrogel injection site, preferably for Matrigel as the hydrogel, in which drops of Matrigel were introduced into the second flow channel for encapsulating the PSC aggregates (3) arriving in single file. The encapsulated PSC aggregates were guided through the third flow channel and deposited into vessels, herein each encapsulated PSC aggregate with some of the first medium was deposited into a separate vessel. Medium changes in the vessels are e.g. performed by withdrawal of medium from the vessels and adding another medium to the vessels. Medium can be withdrawn from vessels and added to vessels by pipetting, which may be automated (4). The organoids that are produced by the differentiation under static conditions in the sequence of media added to and removed from the vessels can e.g. be used for screening (5) of the effect of compounds onto the organoids.

Example 1: Production of cardiac organoids

HES3 MIXL1-GFP cells, and in another batch, HES3 NKX2.5-eGFP cells were cultivated in a stirred tank bioreactor in E8 medium as a first medium, having the composition of DMEM/F12, L-ascorbic acid-2-phosphate magnesium (64 mg/L), sodium selenium (14 $\mu$g/L), FGF2 (100 $\mu$g/L), insulin (19.4 mg/L), NaHCO$_3$ (543 mg/L) and transferrin (10.7 mg/L), TGF$\beta$1 (2 $\mu$g/L) or NODAL (100 $\mu$g/L). Osmolarity of all media was adjusted to 340 mOsm at pH7.4, in order to provide for pluripotency of the ESC. Preferably, the first medium is supplemented with ROCK inhibitor, e.g. 10 $\mu$M ROCK inhibitor (Rho-associated-kinase inhibitor).

For the bioreactor (DASbox® Mini Bioreactor System, Eppendorf, vessel diameter of 0.064 m, impeller diameter of 0.034 m, impeller width of 0.01 m, number of blades of 8, blade angle of 60°), the speed of the stirrer could be pre-determined as the highest speed possible at which previously produced PSC aggregates suspended in cell culture medium were not disrupted, as this speed also effectively distributed dissolved oxygen throughout the medium. Alternatively, lower speeds were tested, e.g. reduced by 5% or 10% from the highest speed possible. For this bioreactor, for a volume of medium of 0.15 L, the optimum stirrer speed was determined to 50 rpm. Oxygen, CO$_2$, air and nitrogen were provided by the controlled gassing system incorporated in the bioreactor system enabling both control of dissolved oxygen and monitoring of dissolved oxygen by an oxygen probe of the bioreactor system. The bioreactor system contained the OxyFerm FDA (Hamilton, USA) as a dissolved oxygen probe.

At 2-4 days of cultivation, sphere-shaped PSC aggregates having a size of 530 to 890 $\mu$m diameter were produced and the medium including the PSC aggregates was removed from the bioreactor.

The PSC aggregates were encapsulated in Matrigel, by carefully depositing single PSC aggregates in Matrigel in a U-shaped well, serving as a cultivation vessel, of a 96-well cell culture plate into which 15 to 30 $\mu$L Matrigel were deposited beforehand, preferably with subsequent incubation for 45 to 60 min under cell culture conditions in order to solidify the hydrogel, followed by addition of cultivation medium. In a preferred embodiment, the PSC aggregates were encapsulated in Matrigel using the process as generally described with reference to Fig. 1, and depositing the hydrogel-encapsulated PSC aggregates singly in wells of a 96-well cell culture plate.

Preferably, 80 to 150 $\mu$L of the first medium was added to the wells, each containing one hydrogel-encapsulated PSC aggregate, with incubation for at least 1 day or at least two days, preferably up to 3 days, preferably for 36 to 60 h.

For differentiation, after removal of the first medium, a second medium is added, containing a first differentiation factor to activate the WNT pathway. The first differentiation factor having activity to induce the WNT pathway preferably is an inhibitor of GSK3beta (glycogen synthase kinase 3 beta), and preferably has no effect or cross-reactivity on CDKs (cyclin-dependent kinases). A preferred first differentiation factor is CHIR99021 (CHIR, 6-[[2-[[4-(2,4-dichlorophenyl)-5-(5-methyl-1H-imidazol-2-yl)-2 pyrimidinyl]amino]ethyl]amino]-3-pyridinecarbonitrile), e.g. at 7.5$\mu$M, e.g. using 200$\mu$L per well. The second medium preferably is RPMI medium containing B27 supplement without insulin and it additionally contains the first differentiation factor. Incubation under cell culture conditions is for at least 6 h, e.g. for up to 3 d, preferably for 12 to 48 h, e.g. for 24h.

Preferably, subsequently, the second medium containing the first differentiation factor is removed, preferably at 24h after adding this second medium, and a third cell culture medium is added, which does not contain a differentiation factor, e.g. no first differentiation factor, and incubating under cell culture conditions, e.g. for 1 d.

The third medium preferably is free from insulin, e.g. RPMI medium containing B27 supplement (RB) but without insulin (RB-). Generally preferably, in the process, only a first differentiation factor having activity to activate the WNT pathway only is added, e.g. contained in a second cell culture medium, and the process, especially, is devoid of adding a differentiation factor having activity to activate another differentiation pathway than the WNT pathway, e.g. devoid of adding BMP4, Rho kinase inhibitor, activin-A and IWR-1.

Subsequently, the medium is removed from the cell aggregate and a fourth cell culture medium is added, which contains a second differentiation factor which inhibits the WNT2 pathway, and preferably the fourth medium contains no insulin. The fourth medium can be RPMI medium containing B27 supplement but without insulin. The second differentiation factor preferably is an inhibitor of the WNT pathway activator Porcupine, e.g. IWP2 (Inhibitor of WNT Production-2, CAS No. 686770-61-6), e.g. at a concentration of 5μM, e.g. using 100μL to 300 μL, e.g. up to 200 μL medium, per well. Incubation under cell culture conditions is for at least 1 d or at least 2 d, preferably for 46 to 50 h, e.g. for 48 h.

Preferably after 2 d incubation, the fourth medium is removed and a fifth medium is added to each well, which medium does not contain a first nor a second inhibitor. The fifth medium preferably contains no insulin. The fifth medium can e.g. be RPMI medium containing B27 supplement without insulin. The volume of the fifth medium can e.g. be 100 μL to 300 μL, e.g. 150 to 250 μL, preferably 200 μL per well. RPMI medium is RPMI1640 (Inorganic Salts: Calcium nitrate * 4H$_2$O (0.1 g/L), magnesium sulfate (0.04884 g/L), potassium chloride (0.4 g/L), sodium bicarbonate (2 g/L), sodium chloride (6 g/L), sodium phosphate dibasic (0.8 g/L); Amino Acids: L-alanyl-L-glutamine (0 g/L), L-arginine (0.2 g/L), L-asparagine (0.05 g/L), L-aspartic acid (0.02 g/L), L-cystine * 2HCl (0.0652 g/L), L-glutamic acid (0.02 g/L), glycine (0.01 g/L), L-histidine (0.015 g/L), hydroxy-L-proline (0.02 g/L), L-isoleucine (0.05 g/L), L-leucine (0.05 g/L), L-lysine * HCl (0.04), L-methionine (0.015 g/L), L-phenylalanine (0.015 g/L), L-proline (0.02 g/L), L-serine (0.03 g/L), L-threonine (0.02 g/L), L-tryptophan (0.005 g/L), L-tyrosine * 2Na * 2H$_2$O (0.02883 g/L), L-valine (0.02 g/L); Vitamins: D-biotin (0.0002 g/L), choline chloride (0.003 g/L), folic acid (0.001 g/L), myo-inositol (0.035 g/L), niacinamide (0.001 g/L), p-aminobenzoic acid (0.001 g/L), D-panthothenic acid (hemicalcium) (0.00025 g/L), pyridoxine * HCl (0.001 g/L), riboflavin (0.0002 g/L), thiamine * HCl (0.001 g/L), vitamin B12 (0.000005 g/L); D-glucose (2 g/L), glutathione (0.001 g/L), phenol red * Na (0.0053 g/L); L-Glutamine (0.3 g/L), sodium bicarbonate (0 g/L)). Incubation under cell culture conditions is for at least 1 d or at least 2 d, preferably for 2 days.

Subsequently, the fifth medium is removed and replaced by a sixth medium which contains insulin, e.g. RPMI medium containing B27 supplement (containing insulin). This medium is preferably replaced by fresh cell culture medium which preferably contains insulin.

The process for producing the cardiac organoids has the advantage that only one type of cells, namely PSC, e.g. iPSC or ESC, can be used in the process and that during the process the PSC, e.g. iPSC or ESC, differentiate and self-organize into a three-dimensional structure which comprises adjoining layers comprising or consisting of different cell types (e.g. cardiomyocytes, endothelial cells, endodermal cells, mesenchymal cells), e.g. without mechanically manipulating cells into a specific layered structure, and without initially providing different cell types.

Generally, all media can contain anti-bacterial agents, e.g. penicillin and/or streptomycin for the prevention of bacterial contaminations.

Fig. 2 and Fig 3 show photographs (scale bar is 500 μm) of cardiac organoids from HES3 MIXL1-GFP at day 1 (d1) of differentiation, and for HES3 NKX2.5-eGFP cells at day 10 (d10) of differentiation as described above (Bioreactor protocol), and produced according to the process of WO 2019/174879 A1 as a comparison (Standard protocol).

The results show that organoids can be produced by differentiating PSC aggregates produced in a stirred tank bioreactor by encapsulating in hydrogel, followed by differentiation induced by differentiation media under static cell culture conditions.

Example 2: Production of blood producing cardiac organoids

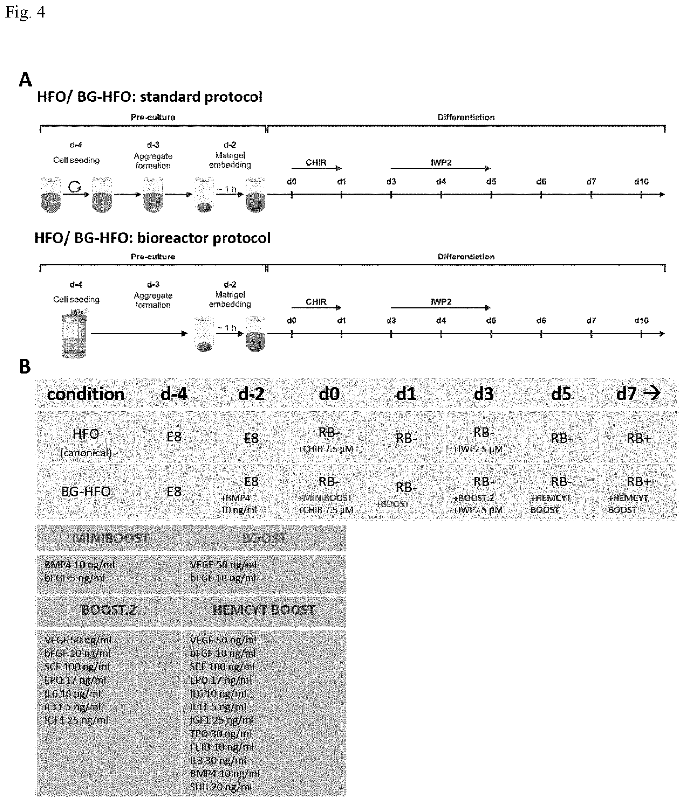

As a modification of the general production method as detailed in Example 1, the media were supplemented with specific cytokines at specific time-points, resulting in hematopoietic differentiation, as schematically shown in Fig. 4. In addition to the formation of hemogenic endothelium (HE), which is already present in the cardiac organoids produced according to Example 1, the addition of the specific cytokines resulted in production of blood-producing cardiac organoids, which are specified by the induction of rounded hematopoietic progenitors (HEM) expressing the markers CD43 and CD45.

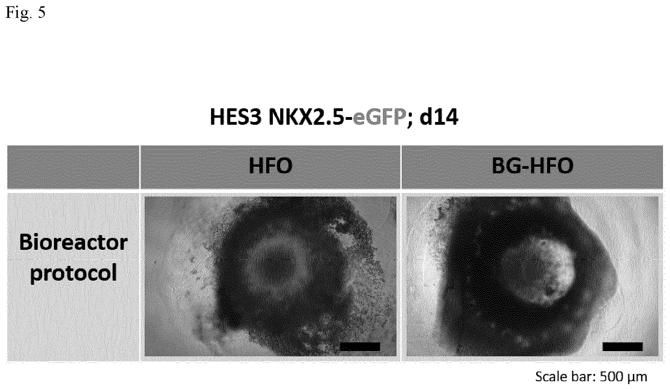

Fig. 5 shows pictures (scale bar is 500 μm) of cardiac organoids produced from HES3 NKX2.5-eGFP cells in the stirred tank bioreactor with the differentiation according to Example 1 (HFO) and with the additional specific cytokines (BG-HFO) on day 14 of differentiation (d14). It can be seen that BG-HFOs can be produced accordingly to HFOs by differentiating PSC aggregates produced in a stirred tank bioreactor by encapsulating in a hydrogel, followed by differentiation under static cell culture conditions. BG-HFOs show a similar NKX2.5-eGFP layered pattern as HFOs.

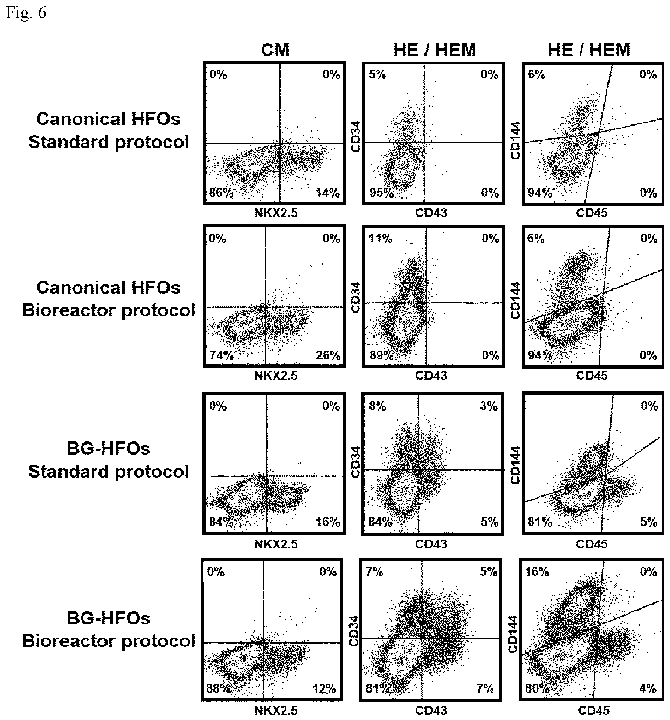

Quantitative flow cytometry analyses (Fig. 6) showed that the percentage of cells positive for the endothelial markers CD31, CD144 and CD34 were increased in the organoids differentiated in presence of the specific cytokines (BG-HFOs) compared to those of Example 1 (HFOs). Additionally, cells positive for the hematopoietic progenitor markers CD43 and CD45 were detected in BG-HFOs. The amount of endothelial and hematopoietic cells in BG-HFOs and HFOs was similar between the stationary (Standard protocol) and the automated approach using a stirred-tank bioreactor according to the invention (Bioreactor protocol).

Example 3: Production of lung-progenitor cells containing organoids (HFOs)

Aggregates were produced in a stirred-tank bioreactor from HES3 NKX2.5-eGFP cells according to Example 1, removed from the bioreactor and embedded in Matrigel. The Matrigel-embedded PSC aggregates were cultivated in medium containing CHIR and IWP2 until d7, and from d7 until d14, the Matrigel-embedded aggregates were cultured in lung differentiation medium, which consists of Lung basal medium (Knockout DMEM, 5% Knockout Serum Replacement, 1% L-glutamine, 1% non-essential amino acids, 0.46 mM 1-thioglycerol) supplemented with 3 μM CHIR, 10 ng/mL FGF10, 10 ng/mL BMP4), with daily medium changes for differentiation. Analysis was performed on d14.

In the embryo, anterior foregut endoderm gives rise to different organs, e.g. the lung. In the present exemplary process for producing HFOs from PSC aggregates generated in a stirred-tank bioreactor and after embedding in Matrigel and cultivation in lung differentiation medium, it was found that lung progenitor cells form in the inner core of the organoids as cells that present the lung progenitor marker NKX2.1.

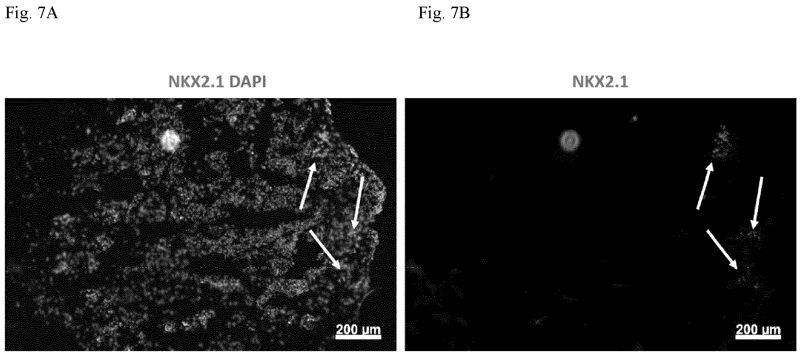

Fig. 7A (scale bar 200 μm) shows a cryosection of a lung-progenitor cell containing cardiac organoid with immunostaining for NKX2.1 (Cy3-labelled anti-NKX2.1 antibody obtained from Jackson ImmunoResearch/Dianova) and DAPI staining, and Fig. 7B (scale bar 200 μm) shows the same cryosection with illumination for NKX2.1 only. The arrows point to NKX2.1-positive lung progenitor cells that are arranged in the inner core of the organoid.

The examples show that production of PSC aggregates under controlled conditions in a stirred-tank bioreactor with subsequent embedding of the aggregates in hydrogel and cultivating the hydrogel-embedded aggregates in medium containing differentiation factors according to the invention can generate cardiac organoids (HFOs), which in addition to cardiac cells contain lung progenitor cells. Therein, during the production by cultivation in a stirred-tank bioreactor, medium that is devoid of differentiation factors is used, and optionally subsequently changing the medium within the stirred-tank bioreactor for medium containing differentiation factors and cultivation within the stirred-tank bioreactor, with subsequent embedding of the aggregates in hydrogel, preferably Matrigel, and cultivation, preferably under static conditions, in medium containing differentiation factors, and optionally subsequently cultivating in medium devoid of differentiation factors.

Example 4: Production of brain organoids with embedding aggregates in hydrogel

Human ESC were produced in a stirred tank bioreactor as described in Example 1, culturing the cells for 2 days in enriched E8 medium (for enriched E8 medium, the standard E8 medium was enriched with 0.1% Pluronic™ F-68 non-ionic surfactant (10%) + 2 mM (4.5 mM in total) L-glutamine, + 3 g/L glucose (https://star-protocols.cell.com/protocols/1227 see E8 full feed medium I (for perfusion feeding from day 1 - day 4)). Subsequently, hPSC aggregate differentiation was performed according to Lancaster et al. (Nature, 2013) by static culture of the aggregates in a 24-well ultra-low-attachment plate for 5 days in neural induction medium (DMEM/F12, 1:100 N2 supplement, 1:100 Glutamax, 1:200 MEM-NEAA, and 1 μg/ml Heparin), followed by Matrigel embedding and culture in a U-shaped ultra-low attachment 96-well plate (as described in Example 1) for 4 days in neural differentiation medium (1:1 mixture of DMEM/F12 and Neurobasal medium containing 1:200 N2 supplement, 1:100 B27 supplement without vitamin A, 3.5 μl/L 2-mercaptoethanol, 1:4000 insulin, 1:100 Glutamax, and 1:200 MEM-NEAA).

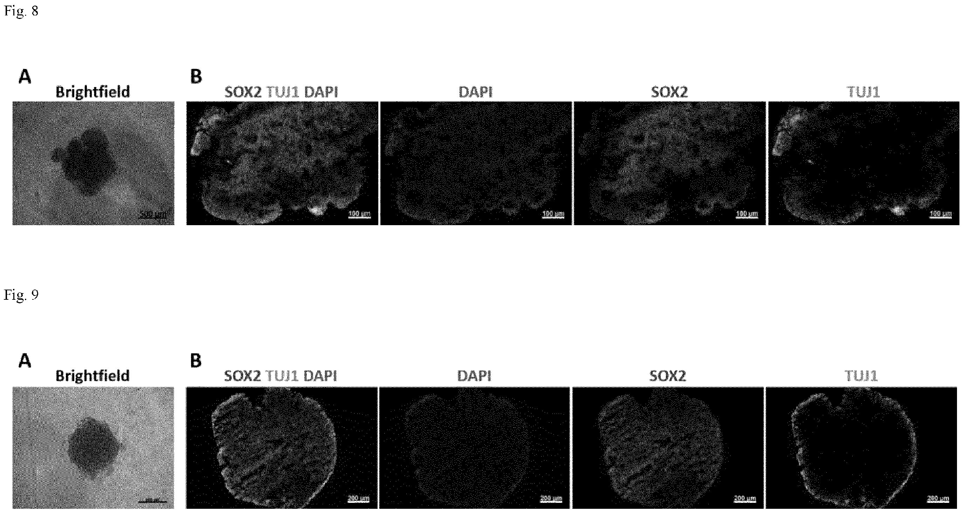

The resulting brain organoids showed the typical neural epithelium morphology (Fig. 8A: Brightfield image of a brain organoid, Fig. 8B: Cryosection of a brain organoid stained for the neuronal markers SOX2 and TUJ1 as well as DAPI to stain nuclei) and expressed the neuronal markers SOX2 and TUJ1.

Example 5: Production of brain organoids with differentiation in stirred tank bioreactor

In another embodiment for producing brain organoids, the hESC aggregates that were produced by cultivation in a stirred-tank reactor in medium devoid of differentiation factors for 2 to 3 days remained in the bioreactor and were cultured for another 5 days in neural induction medium (DMEM/F12, 1:100 N2 supplement, 1:100 Glutamax, 1:200 MEM-NEAA, and 1 μg/ml heparin). Subsequent differentiation was performed manually by Matrigel embedding of the aggregates and static culture in a U-shaped ultra-low attachment 96-well plate (as described in Example 1) for 4 days in neural differentiation medium (1:1 mixture of DMEM/F12 and neurobasal medium containing 1:200 N2 supplement, 1:100 B27 supplement without vitamin A, 3.5 μl/L 2-mercaptoethanol, 1:4000 insulin, 1:100 Glutamax, and 1:200 MEM-NEAA).

The resulting brain organoids showed the typical neural epithelium morphology and expressed the neuronal markers SOX2 and TUJ1 (Fig. 9A: Brightfield image of a brain organoid, Fig. 9B: Cryosection of a brain organoid stained for the neuronal markers SOX2 and TUJ1 as well as DAPI to stain nuclei).

This shows that the production process is suitable for producing different organoid types, which can be derived from all three germ layers, namely from mesoderm and endoderm as present in cardiac organoids and blood generating cardiac organoids (BG-HFO), as well as ectoderm in brain organoids.

**Claims**

1. Process for producing organoids comprising

   a) cultivating pluripotent stem cells (PSC) in cell culture medium devoid of differentiation factors with agitation of the medium under conditions suitable for producing PSC aggregates,
   b) removing PSC aggregates suspended in cell culture medium from the bioreactor,
   c) encapsulating PSC aggregates in biocompatible hydrogel to produce separate hydrogel-encapsulated PSC aggregates,
   d) depositing hydrogel-encapsulated PSC aggregates in cultivation vessels, preferably each hydrogel-encapsulated PSC aggregate in a separate cultivation vessel, and
   e) incubating the hydrogel-encapsulated PSC aggregates in cultivation vessels under static conditions in medium containing at least one differentiation factor,

2. Process according to claim 1, **characterized in that** in step c) prior to encapsulating PSC aggregates, PSC aggregates having a pre-determined shape and/or size are selected.

3. Process according to one of the preceding claims, **characterized in that** in step c), the PSC aggregates are separately encapsulated in biocompatible hydrogel.

4. Process according to one of the preceding claims, **characterized in that** in step a) cultivating with agitation is cultivating the PSC in a stirred tank bioreactor.

5. Process according to one of the preceding claims, **characterized in that** prior to depositing hydrogel-encapsulated PSC aggregates, hydrogel-encapsulated PSC aggregates having a pre-determined shape and/or size are selected.

6. Process according to one of the preceding claims, **characterized in that** in step d), the hydrogel-encapsulated PSC aggregates are each deposited in separate cultivation vessels.

7. Process according to one of the preceding claims, **characterized in that** in step a) the conditions comprise oxygen supply and an agitation in a cylindrical bioreactor with a constant volumetric power input applied to the impeller of P/V = 0.13 to 1.0 W/m$^3$ of liquid contained in the bioreactor, calculated with equation 1

$$\text{equation 1:}$$

$$\frac{P}{V} = \frac{N^3 \times (D \times w \times b_n \div D_V \times \sin\theta)^5 \times \rho}{V}$$

wherein

P = Power [W]
V = liquid volume [m3]
N = impeller rotational speed [s-1]
D = impeller diameter [m]
w = impeller width [m]
bn = number blades of impeller [-]
DV = diameter of bioreactor [m]
θ = blade angle [°]
ρ = liquid density [kg/m3],

wherein the liquid density is determined for the cell-free fraction of the liquid contained in the bioreactor.

8. Process according to one of the preceding claims, **characterized in that** PSC aggregates are encapsulated separately in biocompatible hydrogel to produce separate hydrogel-encapsulated PSC aggregates in a flow channel by flowing the PSC aggregates separately in the flow channel, and continuously or stepwise introducing hydrogel into the flow channel.

9.  Process according to one of the preceding claims, **characterized in that** in step a) the PSC are cultivated in medium devoid of differentiation factors for 2 to 3 days for producing PSC aggregates, and subsequently the PSC aggregates are cultivated in the stirred-tank reactor in medium containing differentiation factors, preferably under the same agitation and oxygenation conditions of step a).

10. Process according to one of the preceding claims, **characterized in that** the media for incubation of hydrogel-encapsulated PSC aggregates during differentiation in addition to cardiac differentiation factors contain specific cytokines, which are added at day-2 of encapsulation in hydrogel: BMP4 to 10 ng/ml, at day 0: BMP4 to 10 ng/ml and bFGF to 5 ng/ml, at day 1: VEGF to 50 ng/ml and bFGF to 10 ng/ml in medium devoid of further differentiation factors, at day 3 VEGF to 50 ng/ml, bFGF to 10 ng/ml, SCF to 100 ng/ml, EPO to 17 ng/ml, IL6 to 10 ng/ml, IL11 to 5 ng/ml, and IGF1 to 25 ng/ml, to insulin-free medium at day 5 and to insulin-containing medium at day 7 VEGF to 50 ng/ml, bFGF to 10 ng/ml, SCF to 100 ng/ml, EPO to 17 ng/ml, IL6 to 10 ng/ml, IL11 to 5 ng/ml, IGF1 to 25 ng/ml, TPO to 30 ng/ml, FLT3 to 10 ng/ml, IL3 to 30 ng/ml, BMP4 to 10 ng/ml, and SHH to 20 ng/ml.

11. Process according to one of claims 1 to 10, **characterized in that** in step e) the hydrogel-encapsulated PSC aggregates are differentiated into organoids containing lung progenitor cells by cultivating hydrogel-embedded PSC aggregates in differentiation medium containing CHIR, FGF10 and BMP4.

12. Process according to one of claims 1 to 7, **characterized in that** in step a) PSC aggregates are differentiated into brain organoids by culturing the aggregates in neural induction medium before encapsulation in Matrigel, and e) culturing the hydrogel-encapsulated aggregates in neural differentiation medium.

13. Process for producing brain organoids comprising

    a) cultivating pluripotent stem cells (PSC) in cell culture medium devoid of differentiation factors in a stirred tank bioreactor under conditions suitable for producing PSC aggregates, the conditions comprising oxygen supply and agitation in a cylindrical bioreactor with a constant volumetric power input applied to the impeller of P/V = 0.13 to 1.0 W/m$^3$ of liquid contained in the bioreactor, calculated with equation 1

$$\text{equation } 1:$$

$$\frac{P}{V} = \frac{N^3 \times (D \times w \times b_n \div D_V \times \sin\theta)^5 \times \rho}{V}$$

    wherein

    P = Power [W]
    V = liquid volume [m3]
    N = impeller rotational speed [s-1]
    D = impeller diameter [m]
    w = impeller width [m]
    bn = number blades of impeller [-]
    DV = diameter of bioreactor [m]
    $\theta$ = blade angle [°]
    $\rho$ = liquid density [kg/m3],

    wherein the liquid density is determined for the cell-free fraction of the liquid contained in the bioreactor,
    b) while continuing cultivating the PSC aggregates under these conditions, differentiating the PSC aggregates by culturing the PSC aggregates for another 5 days in neural induction medium in the bioreactor,
    c) embedding of the aggregates in Matrigel,
    d) and cultivating the Matrigel-embedded aggregates under static conditions in neural differentiation medium.

14. Organoids, obtainable by a process according to one of claims 1 to 10, **characterized by** containing blood generating cells and by presenting the cardiac marker NKX2.5, the endothelial markers CD34 and CD144, and the hematopoietic markers CD43 and CD45, and optionally contain lung progenitor cells presenting NKX2.1.

15. Brain organoids, obtainable by a process according to one of claims 11 to 13, **characterized by** neural epithelium morphology and expressing the neuronal markers SOX2 and TUJ1

Fig. 1

Fig. 2

**HES3 MIXL1-GFP; d1**

Standard protocol          Bioreactor protocol

Fig. 3

**HES3 NKX2.5-eGFP; d10**

Standard protocol          Bioreactor protocol

Fig. 4

**A**

**HFO/ BG-HFO: standard protocol**

**HFO/ BG-HFO: bioreactor protocol**

**B**

| condition | d-4 | d-2 | d0 | d1 | d3 | d5 | d7 → |
|---|---|---|---|---|---|---|---|
| HFO (canonical) | E8 | E8 | RB- +CHIR 7.5 µM | RB- | RB- +IWP2 5 µM | RB- | RB+ |
| BG-HFO | E8 | E8 +BMP4 10 ng/ml | RB- +MINIBOOST +CHIR 7.5 µM | RB- +BOOST | RB- +BOOST.2 +IWP2 5 µM | RB- +HEMCYT BOOST | RB+ +HEMCYT BOOST |

| MINIBOOST | BOOST |
|---|---|
| BMP4 10 ng/ml<br>bFGF 5 ng/ml | VEGF 50 ng/ml<br>bFGF 10 ng/ml |

| BOOST.2 | HEMCYT BOOST |
|---|---|
| VEGF 50 ng/ml<br>bFGF 10 ng/ml<br>SCF 100 ng/ml<br>EPO 17 ng/ml<br>IL6 10 ng/ml<br>IL11 5 ng/ml<br>IGF1 25 ng/ml | VEGF 50 ng/ml<br>bFGF 10 ng/ml<br>SCF 100 ng/ml<br>EPO 17 ng/ml<br>IL6 10 ng/ml<br>IL11 5 ng/ml<br>IGF1 25 ng/ml<br>TPO 30 ng/ml<br>FLT3 10 ng/ml<br>IL3 30 ng/ml<br>BMP4 10 ng/ml<br>SHH 20 ng/ml |

Fig. 5

HES3 NKX2.5-eGFP; d14

| | HFO | BG-HFO |
|---|---|---|
| Bioreactor protocol | | |

Scale bar: 500 μm

Fig. 6

Fig. 7B

NKX2.1

Fig. 7A

NKX2.1 DAPI

Fig. 8

**A**
**Brightfield**

**B**
SOX2 TUJ1 DAPI

DAPI

SOX2

TUJ1

Fig. 9

**A**
**Brightfield**

**B**
SOX2 TUJ1 DAPI

DAPI

SOX2

TUJ1

EP 4 310 177 A1

Europäisches
Patentamt

European
Patent Office

Office européen
des brevets

## EUROPEAN SEARCH REPORT

**Application Number**

EP 22 18 5869

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/174879 A1 (MEDIZINISCHE HOCHSCHULE HANNOVER [DE]) 19 September 2019 (2019-09-19) | 14 | INV. C12N5/0735 C12N5/077 |
| Y | * claim 1; examples 1, 6 * | 1-6 | C12N5/071 C12N5/079 |
| X | DRAKHLIS LIKA ET AL: "Generation of heart-forming organoids from human pluripotent stem cells", NATURE PROTOCOLS, NATURE PUBLISHING GROUP, GB, vol. 16, no. 12, 10 November 2021 (2021-11-10), pages 5652-5672, XP037634420, ISSN: 1754-2189, DOI: 10.1038/S41596-021-00629-8 [retrieved on 2021-11-10] | 14 | |
| Y | * page 5654; figure 2 * | 1-6 | |
| X | DRAKHLIS LIKA ET AL: "Human heart-forming organoids recapitulate early heart and foregut development", NATURE BIOTECHNOLOGY, NATURE PUBLISHING GROUP US, NEW YORK, vol. 39, no. 6, 8 February 2021 (2021-02-08), pages 737-746, XP037612771, ISSN: 1087-0156, DOI: 10.1038/S41587-021-00815-9 [retrieved on 2021-02-08] | 14 | |
| Y | * page 737 - page 739; figure 1 * | 1-6 | |
| X | US 2018/298330 A1 (BOLOGNIN SILVIA [LU] ET AL) 18 October 2018 (2018-10-18) | 15 | |
| Y | * paragraph [[0130]]; claim 1; example 1 * | 1-6 | |

**TECHNICAL FIELDS SEARCHED (IPC)**

C12N

-/--

The present search report has been drawn up for all claims

| Place of search | Date of completion of the search | Examiner |
|---|---|---|
| Munich | 31 December 2022 | Paresce, Donata |

## EUROPEAN SEARCH REPORT

Application Number

EP 22 18 5869

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2017/198256 A1 (LIPKE ELIZABETH A [US] ET AL) 13 July 2017 (2017-07-13) | 14 | |
| Y | * page 11, paragraph [0114] – paragraph [0118] * | 1-6 | |
| Y | SART SÉBASTIEN ET AL: "Characterization of 3D pluripotent stem cell aggregates and the impact of their properties on bioprocessing", PROCESS BIOCHEMISTRY, vol. 59, 24 May 2016 (2016-05-24), pages 276-288, XP085197609, ISSN: 1359-5113, DOI: 10.1016/J.PROCBIO.2016.05.024 * figure 3 * | 1-6 | |
| Y | HOFER MORITZ ET AL: "Engineering organoids", NATURE REVIEWS MATERIALS, NATURE PUBLISHING GROUP UK, LONDON, vol. 6, no. 5, 19 February 2021 (2021-02-19), pages 402-420, XP037449291, DOI: 10.1038/S41578-021-00279-Y [retrieved on 2021-02-19] * figure 2 * | 1-6 | TECHNICAL FIELDS SEARCHED (IPC) |
| A | WO 2017/160234 A1 (AGENCY SCIENCE TECH & RES [SG]; NAT UNIV SINGAPORE [SG]) 21 September 2017 (2017-09-21) * figure 1 * | 1-15 | |

The present search report has been drawn up for all claims

| Place of search | Date of completion of the search | Examiner |
|---|---|---|
| Munich | 31 December 2022 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 5869

This annex lists the patent family members relating to the patent documents cited in the above-mentioned European search report.
The members are as contained in the European Patent Office EDP file on
The European Patent Office is in no way liable for these particulars which are merely given for the purpose of information.

31-12-2022

| Patent document cited in search report | Publication date | Patent family member(s) | Publication date |
|---|---|---|---|
| WO 2019174879 A1 | 19-09-2019 | CA 3093561 A1 | 19-09-2019 |
| | | CN 112041429 A | 04-12-2020 |
| | | EP 3540046 A1 | 18-09-2019 |
| | | EP 3765599 A1 | 20-01-2021 |
| | | US 2021017496 A1 | 21-01-2021 |
| | | WO 2019174879 A1 | 19-09-2019 |
| US 2018298330 A1 | 18-10-2018 | EP 3359649 A1 | 15-08-2018 |
| | | JP 2018531011 A | 25-10-2018 |
| | | LU 92845 B1 | 02-05-2017 |
| | | US 2018298330 A1 | 18-10-2018 |
| | | WO 2017060884 A1 | 13-04-2017 |
| US 2017198256 A1 | 13-07-2017 | US 2015132847 A1 | 14-05-2015 |
| | | US 2017198256 A1 | 13-07-2017 |
| | | US 2019284534 A1 | 19-09-2019 |
| | | US 2022403338 A1 | 22-12-2022 |
| WO 2017160234 A1 | 21-09-2017 | CN 109996870 A | 09-07-2019 |
| | | EP 3430132 A1 | 23-01-2019 |
| | | SG 10201913687V A | 30-03-2020 |
| | | SG 11201807487W A | 27-09-2018 |
| | | US 2019169576 A1 | 06-06-2019 |
| | | WO 2017160234 A1 | 21-09-2017 |

For more details about this annex : see Official Journal of the European Patent Office, No. 12/82

**EP 4 310 177 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20170198256 A1
- US 20160271183 A1

- WO 2019174879 A1

### Non-patent literature cited in the description

- **LANCASTER et al.** *Nature,* 2013, vol. 501, 373-379
- **RICHARDS et al.** *Biomaterials,* 2017, 112-123

- **ELLIOTT et al.** NKX2-5(eGFP/w) hESCs for isolation of human cardiac progenitors and cardiomyocytes. *Nature methods,* 2011, vol. 8 (12), 1037-1040
- *CHEMICAL ABSTRACTS,* 686770-61-6
- **LANCASTER et al.** *Nature,* 2013